# EUROPEAN PATENT APPLICATION

(11) **EP 4 008 349 A1**
(43) Date of publication of application: **08.06.2022**
(21) Application number: 20847959.2
(22) Date of filing: 31.07.2020
(51) Int. Cl.: A61K 39/395, A61K 45/00, A61K 48/00, A61P 35/00, A61P 43/00, C07K 14/47, C12N 15/12, C12N 15/861, A61K 33/24, A61K 38/17, A61K 31/282, A61K 31/44, A61K 31/506, A61K 31/517, A61K 31/5377, A61K 31/555, A61K 31/7088, A61K 35/761

(54) **METHOD FOR TREATING LIVER CANCER WITH COMBINED USE OF REIC/DKK-3 GENE AND ANTI-TUMOR AGENT**

(30) Priority: 31.07.2019 JP 2019141677
(71) Applicant: Momotaro-Gene Inc., Okayama-shi, Okayama 700-8558 (JP); National University Corporation Okayama University, Kita-ku, Okayama-shi, Okayama 700-8530 (JP)
(72) Inventor: KUMON Hiromi, Okayama-shi, Okayama 700-0904 (JP); SHIRAHA Hidenori, Okayama-shi, Okayama 700-8530 (JP); OYAMA Atsushi, Okayama-shi, Okayama 700-8530 (JP); UCHIDA Daisuke, Okayama-shi, Okayama 700-8530 (JP); IWAMURO Masaya, Okayama-shi, Okayama 700-8530 (JP); OKADA Hiroyuki, Okayama-shi, Okayama 700-8530 (JP)
(74) Representative: Grünecker Patent- und Rechtsanwälte PartG mbB
(86) International application number: PCT/JP2020/029407
(87) International publication number: WO 2021/020554

(57) **Abstract**

This invention provides a method for treatment of hepatic cancer with the use of an anti-tumor agent in combination with the REIC/Dkk-3 gene. This invention also provides a therapeutic agent for treatment of hepatic cancer used in combination with an anti-tumor agent, which comprises, as an active ingredient, the REIC/Dkk-3 gene.

SEQUENCE LISTING

<110>  Momotaro-Gene Inc.
       National University Corporation Okayama University

<120>  A method of treating cancer by combination of REIC/Dkk-3 gene
       and antitumor agent

<130>  PH-8469-PCT

<150>  JP 2019-141677
<151>  2019-07-31

<160>  7

<170>  PatentIn version 3.5

<210>  1
<211>  1053
<212>  DNA
<213>  Homo sapiens

<220>
<221>  CDS
<222>  (1)..(1053)

<400>  1
atg cag cgg ctt ggg gcc acc ctg ctg tgc ctg cta ctg gcg gcg gcg    48
gtc ccc acg gcc ccc gcg ccc gct ccg acg gcg acc tcg gct cca gtc    96
aag ccc ggc ccg gct ctc agc tac ccg cag gag gag gcc acc ctc aat    144
gag atg ttc cgc gag gtt gag gaa ctg gtg gag gac acg cag cac aaa    192
ttg cgc agc gcg gtg gaa gag atg gag gca gaa gaa gct gct gct aaa    240
gca tca tca gaa gtg aac ctg gca aac tta cct ccc agc tat cac aat    288
gag acc aac aca gac acg aag gtt gga aat aat acc atc cat gtg cac    336
cga gaa att cac aag ata acc aac aac cag gct cga caa atg gtc ttt    384
tca gag aca gtt atc aca tct gtg gga gac gaa gaa ggc aga agg agc    432
cac gag tgc atc atc gac gag gac tgt ggg ccc agc atg tac tgc cag     480
ttt gcc agc ttc cag tac acc tgc cag cca tgc cgg ggc cag agg atg     528
ctc tgc acc cgg gac agt gag tgc tgt gga gac cag ctg tgt gtc tgg     576
ggt cac tgc acc aaa atg gcc acc agg ggc agc aat ggg acc atc tgt     624
gac aac cag agg gac tgc cag ccg ggg ctg tgc tgt gcc ttc cag aga     672
ggc ctg ctg ttc cct gtg tgc ata ccc ctg ccc gtg gag ggc gag ctt     720
tgc cat gac ccc gcc agc cgg ctt ctg gac ctc atc acc tgg gag cta     768
gag cct gat gga gcc ttg gac cga tgc cct tgt gcc agt ggc ctc ctc     816
tgc cag ccc cac agc cac agc ctg gtg tat gtg tgc aag ccg acc ttc     864
gtg ggg agc cgt gac caa gat ggg gag atc ctg ctg ccc aga gag gtc     912
ccc gat gag tat gaa gtt ggc agc ttc atg gag gag gtg cgc cag gag     960
ctg gag gac ctg gag agg agc ctg act gaa gag atg gcg ctg ggg gag     1008
cct gcg gct gcc gcc gct gca ctg ctg gga ggg gaa gag att tag         1053

<210>  2
<211>  350
<212>  PRT
<213>  Homo sapiens

<400>  2

<210>  3
<211>  117
<212>  DNA
<213>  Homo sapiens

<400>  3
atgcagcggc ttggggccac cctgctgtgc ctgctactgg cggcggcggt ccccacggcc      60

cccgcgcccg ctccgacggc gacctcggct ccagtcaagc ccggcccggc tctcagc        117

<210>  4
<211>  234
<212>  DNA
<213>  Homo sapiens

<400>  4
atgcagcggc ttggggccac cctgctgtgc ctgctactgg cggcggcggt ccccacggcc      60

cccgcgcccg ctccgacggc gacctcggct ccagtcaagc ccggcccggc tctcagctac     120

ccgcaggagg aggccaccct caatgagatg ttccgcgagg ttgaggaact ggtggaggac     180

acgcagcaca aattgcgcag cgcggtggaa gagatggagg cagaagaagc tgct           234

<210>  5
<211>  262
<212>  DNA
<213>  Artificial

<220>
<223>  Synthetic

<400>  5
tgactgactg acgtttaaac ccgctgatca gcctcgactg tgccttctag ttgccagcca      60

tctgttgttt gcccctcccc cgtgccttcc ttgaccctgg aaggtgccac tcccactgtc     120

ctttcctaat aaaatgagga aattgcatcg cattgtctga gtaggtgtca ttctattctg     180

gggggtgggg tggggcagga cagcaagggg gaggattggg aagacaatag caggcatgct 240

ggggatgcgg tgggctctat gg 262

<210> 6
<211> 2355
<212> DNA
<213> Artificial

<220>
<223> Synthetic

<400> 6
tctagagcac catgcagcgg cttggggcca ccctgctgtg cctgctgctg gcggcggcgg 60

tccccacggc ccccgcgccc gctccgacgg cgacctcggc tccagtcaag cccggcccgg 120

ctctcagcta cccgcaggag gaggccaccc tcaatgagat gttccgcgag gttgaggaac 180

tgatggagga cacgcagcac aaattgcgca gcgcggtgga agagatggag gcagaagaag 240

ctgctgctaa agcatcatca gaagtgaacc tggcaaactt acctcccagc tatcacaatg 300

agaccaacac agacacgaag gttggaaata ataccatcca tgtgcaccga gaaattcaca 360

agataaccaa caaccagact ggacaaatgg tcttttcaga gacagttatc acatctgtgg 420

gagacgaaga aggcagaagg agccacgagt gcatcatcga cgaggactgt gggcccagca 480

tgtactgcca gtttgccagc ttccagtaca cctgccagcc atgccggggc cagaggatgc 540

tctgcacccg ggacagtgag tgctgtggag accagctgtg tgtctggggt cactgcacca 600

aaatggccac caggggcagc aatgggacca tctgtgacaa ccagagggac tgccagccgg 660

ggctgtgctg tgccttccag agaggcctgc tgttccctgt gtgcacaccc ctgcccgtgg 720

agggcgagct ttgccatgac cccgccagcc ggcttctgga cctcatcacc tgggagctag 780

agcctgatgg agccttggac cgatgccctt gtgccagtgg cctcctctgc cagccccaca 840

gccacagcct ggtgtatgtg tgcaagccga ccttcgtggg gagccgtgac caagatgggg 900

agatcctgct gcccagagag gtccccgatg agtatgaagt tggcagcttc atggaggagg 960

tgcgccagga gctggaggac ctggagagga gcctgactga agagatggcg ctgggggagc 1020

ctgcggctgc cgccgctgca ctgctgggag gggaagagat ttagggggta ccccggctag 1080

atgactaacg tttaaacccg ctgatcagcc tcgactgtgc cttctagttg ccagccatct 1140

gttgtttgcc cctcccccgt gccttccttg accctggaag gtgccactcc cactgtcctt 1200

tcctaataaa atgaggaaat tgcatcgcat tgtctgagta ggtgtcattc tattctgggg 1260

ggtggggtgg ggcaggacag caagggggag gattgggaag acaatagcag gcatgctggg 1320

gatgcggtgg gctctatggc ggagtactgt cctccgcttc ccacgtggcg gagggactgg 1380

ggacccgggc acccgtcctg ccccttcacc ttccagctcc gcctcctccg cgcggacccc 1440

gccccgtccc gacccctccc gggtccccgg cccagccccc tccgggccct cccagcccct 1500

ccccttcctt tccgcggccc cgccctctcc tcgcggcgcg agttttggaa agtccccagg 1560

ctccccagca ggcagaagta tccaaagcat ccatctcaat tagtcagcaa ccaggtgtgg 1620

aaagtcccca ggctccccag caggcagaag tatccaaagc atccatctca attagtcagc 1680

aaccatagtc ccgcccctaa ctccgcccat cccgccccta actccgccca gttccgccca 1740

ttctccgccc catggctgac taattttttt tatttatgca gaggccgagg ccgcctctgc 1800

ctctgagcta ttccagaagt agtgaggagg cttttttgga ggccaaggct tttgcaaaaa 1860

gctccgttac ataacttacg gtaaatggcc cgcctggctg accgcccaac gacccccgcc 1920

cattgacgtc aataatgacg tatgttccca tagtaacgcc aatagggact ttccattgac 1980

gtcaatgggt ggagtattta cggtaaactg cccacttggc agtacatcaa gtgtatcata 2040

tgccaagtac gccccctatt gacgtcaatg acggtaaatg gcccgcctgg cattatgccc 2100

agtacatgac cttatgggac tttcctactt ggcagtacat ctacgtatta gtcatcgcta 2160

ttaccatggt gatgcggttt tggcagtaca tcaatgggcg tggatagcgg tttgactcac 2220

ggggatttcc aagtctccac cccattgacg tcaatgggag tttgttttgg caccaaaatc 2280

aacgggactt tccaaaatgt cgtaacaact ccgccccatt gacgcaaatg ggcggtaggc 2340

gtgttgccgg aattc 2355

<210> 7
<211> 1285
<212> DNA
<213> Artificial

<220>
<223> Synthetic

<400> 7
gtttaaaccc gctgatcagc ctcgactgtg ccttctagtt gccagccatc tgttgtttgc 60

ccctcccccg tgccttcctt gaccctggaa ggtgccactc ccactgtcct ttcctaataa 120

aatgaggaaa ttgcatcgca ttgtctgagt aggtgtcatt ctattctggg gggtggggtg 180

gggcaggaca gcaaggggga ggattgggaa gacaatagca ggcatgctgg ggatgcggtg 240

ggctctatgg cggagtactg tcctccgctt cccacgtggc ggagggactg gtcctccgct 300

tcccacgtgg cggagggact ggggacccgg gcacccgtcc tgccccttca ccttccagct 360

ccgcctcctc cgcgcggacc ccgccccgtc ccgacccctc ccgggtcccc ggcccagccc 420

cctccgggcc ctcccagccc ctccccttcc tttccgcggc cccgccctct cctcgcggcg 480

cgagttttgg aaagtcccca ggctccccag caggcagaag tatccaaagc atccatctca 540

attagtcagc aaccaggtgt ggaaagtccc caggctcccc agcaggcaga agtatccaaa 600

gcatccatct caattagtca gcaaccatag tcccgcccct aactccgccc atcccgcccc 660

taactccgcc cagttccgcc cattctccgc cccatggctg actaattttt tttatttatg 720

cagaggccga ggccgcctct gcctctgagc tattccagaa gtagtgagga ggcttttttg 780

gaggccaagg cttttgcaaa aagctccgtt acataactta cggtaaatgg cccgcctggc 840

tgaccgccca acgacccccg cccattgacg tcaataatga cgtatgttcc catagtaacg 900

ccaataggga ctttccattg acgtcaatgg gtggagtatt tacggtaaac tgcccacttg 960

gcagtacatc aagtgtatca tatgccaagt acgcccccta ttgacgtcaa tgacggtaaa 1020

tggcccgcct ggcattatgc ccagtacatg accttatggg actttcctac ttggcagtac 1080

atctacgtat tagtcatcgc tattaccatg gtgatgcggt tttggcagta catcaatggg 1140

cgtggatagc ggtttgactc acggggattt ccaagtctcc accccattga cgtcaatggg 1200

agtttgtttt ggcaccaaaa tcaacgggac tttccaaaat gtcgtaacaa ctccgcccca 1260

ttgacgcaaa tgggcggtag gcgtg 1285

## Description

### Technical Field

The present invention relates to an anti-hepatic cancer composition.

### Background Art

Chemotherapy using anticancer agents plays a key role in treatment of cancer and, in particular, advanced hepatic cancer. According to the clinical trials of Sorafenib, which is the standard agent for treatment of advanced hepatic cancer, the medial survival period is 15.6 months, which indicates a poor prognosis (Non-Patent Document 1). In addition to Sorafenib, Regorafenib and Lenvatinib were developed and applied in clinical settings. While therapeutic options were increased, non-inferiority of Regorafenib and Lenvatinib to Sorafenib was confirmed. That is, the prognosis has not yet been significantly improved. In addition, Cisplatin has been used for treatment of advanced hepatic cancer, although such agent is not sufficient in terms of the response rate.

The REIC/Dkk-3 gene has been known to be involved in cell immortalization. It has been reported that expression of the gene is inhibited in cancer cells. In addition, it has been reported that the REIC/Dkk-3 gene is used in cancer treatment (Patent Document 1).

### Prior Art Documents

### Patent Documents

Patent Document 1: WO 01/038528

### Non-Patent Documents

Non-Patent Document 1: Llovet J. M. et al., N. Engl. J. Med., 2008; 359 (4): 378-390

### Summary of the Invention

### Objects to Be Attained by the Invention

As described above, it has been difficult to achieve satisfactory therapeutic effects according to conventional chemotherapy for advanced hepatic cancer. According to conventional chemotherapy for advanced hepatic cancer, in general, side effects of anticancer agents are relatively strong. Because of the intolerance to treatment, accordingly, dose reduction or drug withdrawal would become unavoidable.

The present invention provides a method for treatment of cancer using an anti-tumor agent in combination with the REIC/Dkk-3 gene. The present invention also provides a therapeutic agent comprising, as an active ingredient, the REIC/Dkk-3 gene used in combination with the anti-tumor agent.

### Means for Attaining the Objects

The present inventors have conducted concentrated studies concerning effects of hepatic cancer treatment attained with the use of the REIC/Dkk-3 gene in combination with a chemotherapeutic agent, which is an anti-tumor agent.

The present inventors discovered that the use of the REIC/Dkk-3 gene in combination with the anti-tumor agent would induce apoptosis in tumor cells to inhibit the tumor growth. They also discovered that the effects achieved when the REIC/Dkk-3 gene was used in combination with the anti-tumor agent would be significantly higher than the effects achieved when the REIC/Dkk-3 gene or the anti-tumor agent was independently used. This indicates that the use of the REIC/Dkk-3 gene in combination with the anti-tumor agent is very effective for cancer treatment.

Specifically, the present invention is as described below.
[1] A therapeutic agent for treatment of hepatic cancer used in combination with an anti-tumor agent, which comprises, as an active ingredient, the REIC/Dkk-3 gene.
[2] The therapeutic agent according to [1], wherein the REIC/Dkk-3 gene is integrated into an adenovirus vector.
[3] The therapeutic agent according to [1] or [2], wherein the anti-tumor agent is a platinum-containing agent.
[4] The therapeutic agent according to [3], wherein the platinum-containing agent is selected from the group consisting of Cisplatin, Carboplatin, Nedaplatin, Oxaliplatin, Miriplatin, Mitaplatin, Satraplatin, Picoplatin, and Triplatin.
[5] The therapeutic agent according to [1] or [2], wherein the anti-tumor agent is a tyrosine kinase inhibitor.
[6] The therapeutic agent according to [5], wherein the tyrosine kinase inhibitor is at least one member selected from the group consisting of Sorafenib, Regorafenib, Lenvatinib, Cabozantinib, Axitinib, Sunitinib, Pazopanib, and Erlotinib.
[7] The therapeutic agent according to [5], wherein the tyrosine kinase inhibitor targets a vascular endothelial growth factor receptor (VEGFR).
[8] The therapeutic agent according to [7], wherein the VEGFR-targeting tyrosine kinase inhibitor is selected from the group consisting of Sorafenib, Regorafenib, Lenvatinib, Cabozantinib, Axitinib, Sunitinib, and Pazopanib.
[9] The therapeutic agent according to [1] or [2], wherein the anti-tumor agent is an immune checkpoint inhibitor.
[10] The therapeutic agent according to [9], wherein the immune checkpoint inhibitor is a PD-1/PD-L1 immune checkpoint pathway inhibitor.
[11] The therapeutic agent according to any of [1] to [10], which is in the form of a local administration agent.
[12] A kit of combinatorial therapeutic agents for treatment of hepatic cancer, which comprises an anti-tumor agent in combination with the REIC/Dkk-3 gene.
[13] The kit of combinatorial therapeutic agents according to [12], wherein the REIC/Dkk-3 gene is integrated into an adenovirus vector.
[14] The kit of combinatorial therapeutic agents according to [12] or [13], wherein the anti-tumor agent is a platinum-containing agent.
[15] The kit of combinatorial therapeutic agents according to [14], wherein the platinum-containing agent is selected from the group consisting of Cisplatin, Carboplatin, Nedaplatin, Oxaliplatin, Miriplatin, Mitaplatin, Satraplatin, Picoplatin, and Triplatin.
[16] The kit of combinatorial therapeutic agents according to [12] or [13], wherein the anti-tumor agent is a tyrosine kinase inhibitor.
[17] The kit of combinatorial therapeutic agents according to [16], wherein the tyrosine kinase inhibitor is at least one member selected from the group consisting of Sorafenib, Regorafenib, Lenvatinib, Cabozantinib, Axitinib, Sunitinib, Pazopanib, and Erlotinib.
[18] The kit of combinatorial therapeutic agents according to [16], wherein the tyrosine kinase inhibitor targets a vascular endothelial growth factor receptor (VEGFR).
[19] The kit of combinatorial therapeutic agents according to [18], wherein the VEGFR-targeting tyrosine kinase inhibitor is selected from the group consisting of Sorafenib, Regorafenib, Lenvatinib, Cabozantinib, Axitinib, Sunitinib, and Pazopanib.
[20] The kit of combinatorial therapeutic agents according to [12] or [13], wherein the anti-tumor agent is an immune checkpoint inhibitor.
[21] The kit of combinatorial therapeutic agents according to [20], wherein the immune checkpoint inhibitor is a PD-1/PD-L1 immune checkpoint pathway inhibitor.
[22] The kit of combinatorial therapeutic agents according to any of [12] to [21], wherein the REIC/Dkk-3 gene is in the form of a local administration agent.
[23] An anti-hepatic cancer composition comprising an anti-tumor agent and the REIC/Dkk-3 gene.
[24] The anti-hepatic cancer composition according to [23], wherein the REIC/Dkk-3 gene is integrated into an adenovirus vector.
[25] The anti-hepatic cancer composition according to [23] or [24], wherein the anti-tumor agent is a platinum-containing agent.
[26] The anti-hepatic cancer composition according to [25], wherein the platinum-containing agent is selected from the group consisting of Cisplatin, Carboplatin, Nedaplatin, Oxaliplatin, Miriplatin, Mitaplatin, Satraplatin, Picoplatin, and Triplatin.
[27] The anti-hepatic cancer composition according to [23] or [24], wherein the anti-tumor agent is a tyrosine kinase inhibitor.
[28] The anti-hepatic cancer composition according to [27], wherein the tyrosine kinase inhibitor is at least one member selected from the group consisting of Sorafenib, Regorafenib, Lenvatinib, Cabozantinib, Axitinib, Sunitinib, Pazopanib, and Erlotinib.
[29] The anti-hepatic cancer composition according to [27], wherein the tyrosine kinase inhibitor targets a vascular endothelial growth factor receptor (VEGFR).
[30] The anti-hepatic cancer composition according to [29], wherein the VEGFR-targeting tyrosine kinase inhibitor is selected from the group consisting of Sorafenib, Regorafenib, Lenvatinib, Cabozantinib, Axitinib, Sunitinib, and Pazopanib.
[31] The anti-hepatic cancer composition according to [23] or [24], wherein the anti-tumor agent is an immune checkpoint inhibitor.
[32] The anti-hepatic cancer composition according to [31], wherein the immune checkpoint inhibitor is a PD-1/PD-L1 immune checkpoint pathway inhibitor.
[33] The anti-hepatic cancer composition according to any of [23] to [32], wherein the REIC/Dkk-3 gene is in the form of a local administration agent.

This description includes part or all of the contents as disclosed in the description of Japanese Patent Application No. 2019-141677, which is a priority document of the present application.

### Effects of the Invention

By performing local administration of Ad-REIC in addition to conventional chemotherapy against advanced hepatic cancer, therapeutic effects can be enhanced to a significant extent.

By performing local administration of Ad-REIC in combination, a dose of a chemotherapeutic agent can be effectively reduced. As a result, side effects of the chemotherapeutic agent can be alleviated, and a burden imposed on a patient can be reduced to a significant extent.

Ad-REIC has activity of inducing cancer-cell-specific cell death and activity of immune activation. Such anti-cancer effects are different from effects of damaging hepatic cancer cells achieved with the use of conventional chemotherapeutic agents. Specifically, conventional chemotherapeutic agents have been problematic in terms of attenuated effects caused by the development of tolerance to anticancer agents and unresponsiveness to the treatment. When Ad-REIC is used in combination, a dose of a chemotherapeutic agent can be reduced to a significant extent. Thus, the issue of the development of tolerance to anticancer agents can be reduced. In addition, Ad-REIC has the action mechanism that is different from the action mechanism of conventional chemotherapeutic agents. Thus, Ad-REIC is free of a risk of the development of tolerance to the anticancer agent, and the development of tolerance to the chemotherapeutic agent can be effectively suppressed.

### Brief Description of the Drawings

Fig. 1 shows an example of the Ad-REIC/Dkk-3 structure.
Fig. 2 shows the Ad-REIC/Dkk-3 sequence.
Fig. 3 shows the results of REIC detection by immunoblotting.
Fig. 4 shows a lowering in cell viability caused by Ad-REIC.
Fig. 5 shows a lowering in cell viability caused with the use of Ad-REIC in combination with Cisplatin.
Fig. 6 shows the results of immunoblot analysis of the JNK signal transmission pathway in hepatic cancer cells.
Fig. 7 shows effects of tumor growth inhibition achieved by the treatment involving the use of Ad-REIC in combination with Cisplatin in a xenograft mouse model of hepatic cancer.
Fig. 8 shows effects of tumor growth inhibition achieved by the treatment involving the use of Ad-REIC in combination with Cisplatin in an allograft mouse model of hepatic cancer.
Fig. 9 shows effects of tumor growth inhibition achieved by the treatment involving the use of Ad-REIC in combination with an anti-PD-1 antibody in an allograft mouse model of hepatic cancer.
Fig. 10A shows effects of tumor growth inhibition achieved by the treatment involving the use of Ad-REIC in combination with a tyrosine kinase inhibitor (Sorafenib) in an allograft mouse model of hepatic cancer.
Fig. 10B shows effects of tumor growth inhibition achieved by the treatment involving the use of Ad-REIC in combination with a tyrosine kinase inhibitor (Lenvatinib) in an allograft mouse model of hepatic cancer.
Fig. 11 shows a lowering in cell viability caused with the use of Ad-REIC in combination with a tyrosine kinase inhibitor.

### Embodiments of the Invention

Hereafter, the present invention is described in detail.

Combination treatment of hepatic cancer according to the present invention involves the use of the REIC/Dkk-3 gene in combination with anti-tumor agents.

The nucleotide sequence of the REIC/Dkk-3 DNA is shown in SEQ ID NO: 1 in the sequence listing. The amino acid sequence of the REIC/Dkk-3 protein encoded by the REIC/Dkk-3 DNA is shown in SEQ ID NO: 2 in the sequence listing. Any DNA having a homology of at least 85%, preferably at least 90%, further preferably at least 95%, and particularly preferably at least 97% to the nucleotide sequence represented by SEQ ID NO: 1 upon calculation by BLAST (Basic Local Alignment Search Tool at the National Center for Biological Information (NCBI), U.S.A.) or the like (e.g., with the use of default setting parameters) is within the scope of the REIC/Dkk-3 DNA.

A nucleotide fragment of the REIC/Dkk-3 can also be used. Examples of nucleotides each comprising a nucleotide sequence comprising nucleotide 1 to any of nucleotides 117 to 234 in the nucleotide sequence of the REIC/Dkk-3 DNA represented by SEQ ID NO: 1 include a polynucleotide comprising nucleotide 1 to nucleotide 117 (SEQ ID NO: 3) and a polynucleotide comprising nucleotide 1 to nucleotide 234 (SEQ ID NO: 4).

The REIC/Dkk-3 gene can be introduced into a subject in accordance with a conventional technique. Examples of methods of gene transfection into a subject include a method involving the use of a viral vector and a method involving the use of a non-viral vector.

The REIC/Dkk-3 gene can be introduced into a cell or tissue with the use of a recombinant expression vector comprising a gene expression vector, such as plasmid vector, introduced thereinto without the use of the virus.

Examples of typical viral vectors used for gene transfection include adenovirus, adeno-associated virus, and retrovirus vectors. A target gene can be transfected into a cell by introducing a gene of interest into a DNA or RNA virus, such as detoxicated retrovirus, herpesvirus, vaccinia virus, poxvirus, poliovirus, Sindbis virus, Sendai virus, SV40, or human immunodeficiency virus (HIV), and allowing the obtained recombinant virus to infect cells. Use of an adenovirus vector is preferable.

A vector comprises a construct containing the REIC/Dkk-3 gene. A construct containing the REIC/Dkk-3 DNA may adequately comprise a promoter, an enhancer for gene transcription, a poly A signal, a marker gene used for labeling and/or selection of cells transfected with a gene, or the like. In such a case, a conventional promoter can be used.

Such a construct comprises the REIC/Dkk-3 gene, at least 1 first promoter, a poly A addition sequence downstream of the first promoter, and an enhance or a second promoter ligated to a site downstream of the DNA construct.

A promoter comprises a particular nucleotide sequence in DNA that initiates transcription using DNA as a template. In general, promoters comprise common sequences. For example, a prokaryotic organism, such as *E. coli,* generally comprises the sequence TATAATG located at the -10-bp position from the transcription initiation site and the sequence TTGACA located at the -35-bp position therefrom. An eukaryotic organism generally comprises the TATA box located at the -20-bp position therefrom. It is necessary that the expression cassette of the present invention comprises a first promoter upstream of the target gene. The expression cassette of the present invention may comprise a second promoter downstream of the target gene. Promoters used as the first promoter and the second promoter are not particularly limited, and the first promoter may be the same with or different from the second promoter. Specific or selective promoters, such as non-specific promoter that can promote foreign gene expression in any cell or tissue, a tissue- or organ-specific promoter, a tumor-specific promoter, or a development- or differentiation-specific promoter, can be used. For example, a specific promoter can be used as a first promoter, and a non-specific promoter can be used as a second promoter. Examples of promoters that can be used in the present invention include: cancer- or tumor-specific promoters, such as human telomerase reverse transcriptase (hTERT), prostate-specific antigen (PSA), c-myc, and GLUT promoters; ES cell and cancer stem cell-specific promoters, such as OCT3/4 and NANOG promoters; neural stem cell-specific promoters, such as Nestin promoter; cell stress-sensitive promoters, such as HSP70, HSP90, and p53 promoters; hepatic cell-specific promoters, such as albumin promoter; radiation-sensitive promoters, such as TNF-alpha promoter; promoters increasing the infected plasmid copy number, such as SV40 promoter; and proliferative cell-specific promoters, such as EF1-alpha promoter. More specific examples of first promoters that can be used include CMV-i promoter (hCMV + intron promoter), β actin promoter, CAG promoter, and CMV promoter, and a more specific example of a second promoter that can be used is CMV promoter. An origin animal species of a β actin promoter is not particularly limited, and mammalian β actin promoters, such as a human β actin promoter or a chicken actin promoter, can be used. An artificial hybrid promoter, such as the aforementioned CMV-i promoter, can also be used. The CMV-i promoter can be synthesized on the basis of, for example, U.S. Patent No. 5,168,062 or 5,385,839. A promoter may be a part of a core promoter consisting of a minimal sequence having promoter activity. A core promoter is a promoter region having activity of inducing accurate transcription initiation and it may comprise the TATA box. Among the promoters described above, a cancer- and/or tumor-specific promoter, such as hTERT promoter, targets cancer. Such promoters can be thus preferably used for gene therapy or cancer diagnosis based on gene expression.

An origin of a poly A addition sequence (polyadenylation sequence, polyA) is not limited, and examples of such include poly A addition sequences derived from a growth hormone gene, such as a poly A addition sequence derived from a bovine growth hormone gene, a poly A addition sequence derived from a human growth hormone gene, a poly A addition sequence derived from SV40 virus, and a poly A addition sequence derived from a human or rabbit β globin gene. By integrating a poly A addition sequence into an expression cassette, transcription efficiency is increased. The nucleotide sequence of the BGA poly A addition sequence is shown in a sequence after nucleotide 13 of the nucleotide sequence represented by SEQ ID NO: 5.

An enhancer is not limited, provided that it can increase the amount of messenger RNA (mRNA) generated by transcription. An enhancer comprises a nucleotide sequence having effects of accelerating promoter activity. In general, an enhancer is of approximately 100 bp. An enhancer can accelerate transcription regardless of the sequence direction. In the present invention, a single type of an enhancer may be used, a plurality of enhancers of the same type may be used, or a plurality of enhancers of different types may be used in combination. When a plurality of enhancers of different types are used, the order of the use is not limited. For example, CMV enhancer, SV40 enhancer, or telomerase reverse transcriptase (hTERT) enhancer can be used. For example, an enhancer may be composed of a sequence comprising hTERT enhancer, SV40 enhancer, and CMV enhancer ligated in that order.

A plurality of enhancers, such as 1 to 4 enhancers, may be ligated to a site upstream of a DNA construct comprising DNA encoding a protein to be expressed and a poly A addition sequence. While an enhancer to be ligated to an upstream site is not limited, CMV enhancer is preferable. An example is a 4 × CMV enhancer comprising 4 CMV enhancers ligated to each other.

When an enhancer is inserted into a site adjacent downstream of the DNA construct comprising "promoter-target gene to be expressed-poly A addition sequence," a protein of the target gene can be expressed at a higher level compared with a conventional gene expression system.

With the use of the CMV i promoter in combination with the CMV enhancer, in particular, a protein of the target gene can be expressed at a distinctly high level upon insertion of any gene into substantially any of the cells (host cells) with the use of any transfection reagent.

In addition, RU5' may be ligated to a site adjacent upstream of DNA encoding a protein to be expressed. When RU5' is ligated to a site " adjacent upstream," RU5' is directly ligated without the aid of an element having other particular functions. A short sequence may be inserted therebetween as a linker. RU5' is HTLV-derived LTR, which is an element that increases the protein expression level upon insertion thereof (Mol. Cell. Biol., Vol. 8 (1), pp. 466-472, 1988). When RU5' is inserted into a site opposite from the site as reported, effects of a promoter for increasing an expression level by enhancer introduction may be cancelled.

In addition, UAS may be ligated to a site adjacent upstream of the enhancer and/or the promoter. UAS is a region to which the GAL4 gene binds. By inserting the GAL4 gene downstream thereof, a protein expression level is increased.

Further, SV40-ori may be ligated to the uppermost region of the expression cassette. SV40-ori is a region to which the SV40 gene binds. By inserting the SV40 gene, a protein expression level is increased. It is necessary that the elements described above be operably linked to each other. When such elements are operably linked to each other, each of such elements exerts its functions to increase the expression level of the target gene to be expressed.

The DNA construct comprises the REIC/Dkk-3 DNA, a cytomegalovirus (CMV) promoter ligated to a site upstream of the REIC/Dkk-3 DNA, and a poly A addition sequence (polyadenylation sequence, poly A) ligated to a site downstream of the REIC/Dkk-3 DNA. In addition, a sequence in which a telomerase reverse transcriptase (hTERT) enhancer, an SV40 enhancer, and a cytomegalovirus (CMV) enhancer ligated to in that order (3 × enh) is ligated to a site downstream of the poly A addition sequence. Specifically, the DNA construct is a construct in which (i) CMV promoter, (ii) the REIC/Dkk-3 DNA, (iii) poly A addition sequence, and (iv) an enhancer in which the telomerase reverse transcriptase (hTERT) enhancer, the SV40 enhancer, and the CMV enhancer ligated in that order from the 5' terminus.

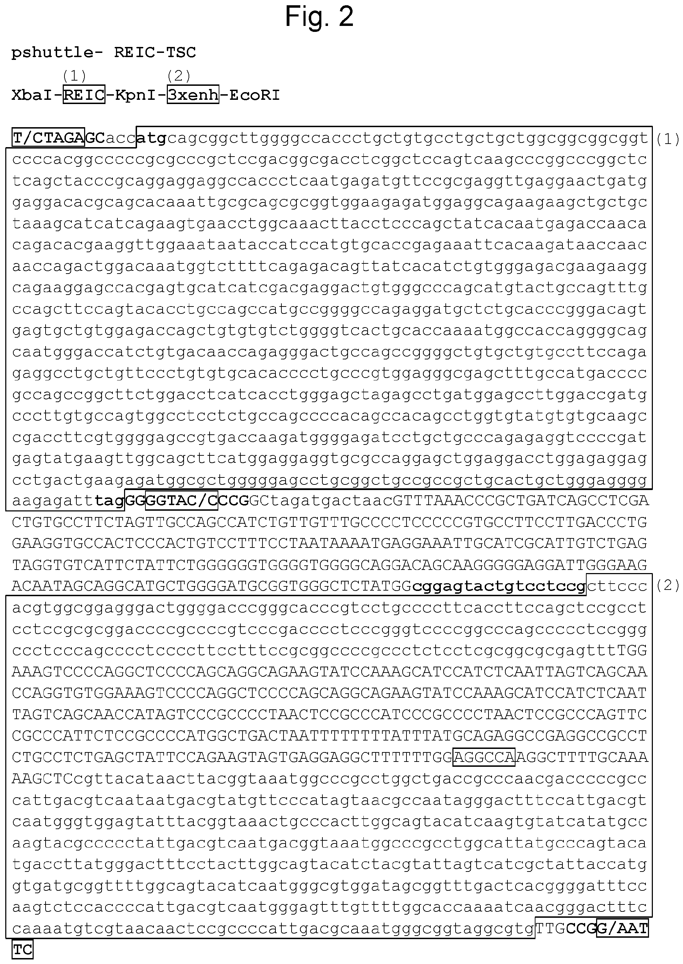

Fig. 2 and SEQ ID NO: 6 show a partial sequence excluding the CMV promoter of the DNA construct including the REIC/Dkk-3 DNA according to the present invention. In Fig. 2, the BGA poly A sequence is inserted into a site between the REIC/Dkk-3 DNA and 3 × enh. The DNA construct comprising the REIC/Dkk-3 DNA according to the present invention has a CMV promoter in a region upstream (on the 5' side) of the sequence represented by SEQ ID NO: 6. SEQ ID NO: 7 shows a nucleotide sequence of a region including BGH poly A and 3 enhancers contained in the above construct. In Fig. 2, regions surrounded by the frames (1) and (2) in the nucleotide sequence respectively indicate a DNA encoding the the REIC/Dkk-3 protein and 3 enhancers (3 × enh).

It is necessary that the elements described above be operably linked to each other. When such elements are operably linked to each other, each of such elements exerts its functions to increase the expression level of the target gene to be expressed.

The expression cassette can be obtained by, for example, inserting the REIC/Dkk-3 DNA into a pShuttle vector (Clonetech) comprising a foreign gene insertion site downstream of a commercially available CMV promoter and the BGA poly A sequence downstream of the foreign gene insertion site; and ligating the telomerase reverse transcriptase (hTERT) enhancer, the SV40 enhancer, and the CMV enhancer in that order to a site downstream of the BGA poly A sequence.

The DNA construct comprising the REIC/Dkk-3 DNA is as described below.
[1] A DNA construct to express the REIC/Dkk-3 protein in which, from the 5' terminus, (i) to (iv) ligated in that order:
   (i) a CMV promoter;
   (ii) REIC/Dkk-3 DNA (a) or (b):
      (a) DNA consisting of the nucleotide sequence represented by SEQ ID NO: 1; or
      (b) DNA having sequence identity of at least 90%, 95%, 97%, or 98% to the nucleotide sequence represented by SEQ ID NO: 1;
   (iii) a poly A addition sequence; and
   (iv) an enhancer in which the telomerase reverse transcriptase (hTERT) enhancer, the SV40 enhancer, and the CMV enhancer ligated in that order.
[2] The DNA construct according to [1], wherein the poly A addition sequence is derived from the bovine growth hormone gene (BGA polyA).
[3] The DNA construct according to [1] or [2], which comprises the nucleotide sequence represented by SEQ ID NO: 6 comprising (ii) the REIC/Dkk-3 DNA, (iii) the poly A addition sequence, and (iv) the enhancer in which the telomerase reverse transcriptase (hTERT) enhancer, the SV40 enhancer, and the CMV enhancer ligated in that order.

The DNA construct can be prepared in accordance with, for example, WO 2011/062298, US Patent Application Publication No. 2012-0309050, WO 2012/161352, or US Patent Application Publication No. 2014-0147917.

In the present invention, an adenovirus vector comprising the REIC/Dkk-3 DNA is referred to as "Ad-REIC" or "Ad-REIC/Dkk-3."

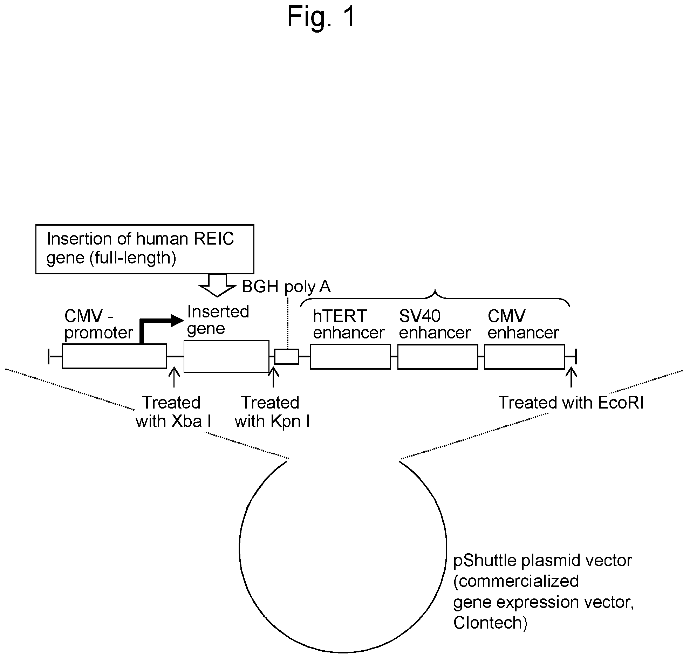

A vector system comprising the DNA construct according to the present invention is referred to as the "Super Gene Expression" (SGE) system, and an adenovirus vector comprising, for example, a DNA construct comprising the REIC/Dkk-3 DNA is referred to as "Ad5-SGE-REIC/Dkk-3." Fig. 1 shows an example of the Ad-REIC/Dkk-3 structure and Fig. 2 shows an example of the Ad-REIC/Dkk-3 sequence.

An adenovirus vector comprising the DNA construct is obtained by introducing the DNA construct into an adenovirus vector and preparing a recombinant adenovirus. A DNA construct can be introduced into an adenovirus by introducing, for example, a DNA construct in the pShuttle vector containing the DNA construct according to the present invention into an adenovirus.

Adenovirus vectors are characterized in that: (1) gene transfection can be carried out in a variety of cells; (2) gene transfection can be efficiently performed even in cells in growth arrest period; (3) they can be concentrated by centrifugation to obtain high-titer viruses (10 to 11 PFU/ml or more); and (4) they are appropriate for use for direct gene transfection into *in vivo* tissue cells.

As an adenovirus vector used for gene therapy, the following vectors have been developed: a second generation adenovirus vector (Lieber, A., et al., J. Virol., 70, 8944, 1996; Mizuguchi, H. & Kay, M. A., Hum. Gene Ther., 10, 2013, 1999) obtained by deleting the E2 or E4 domain in addition to the E1/E3 domain from a first generation adenovirus vector lacking the E1/E3 domain (Miyake, S., et al., Proc. Natl. Acad. Sci. U.S.A., 93, 1320, 1996); and a third generation adenovirus vector (Steinwaerder, D. S. et al., J. Virol., 73, 9303, 1999) almost completely lacking the adenovirus genome (GUTLESS). For transfection of the gene of the present invention, any adenovirus vector may be used without particular limitation.

A recombinant adenovirus vector comprising the DNA construct containing the REIC/Dkk-3 DNA according to the present invention (hereafter, it may be simply referred to as "the adenovirus vector of the present invention") may be administered to a subject, which is a human or other mammalian animals. Thus, a gene for cancer treatment is delivered to hepatic cancer cells in the subject, the gene is expressed in cancer cells, tumor cell growth is inhibited, and effects on cancer treatment are thus exerted.

The adenovirus vector of the present invention can be administered by a method that can be employed in the field of gene therapy, such as intravascular administration-including intravenous or intra-arterial administration, oral administration, intraperitoneal administration, intratracheal administration, intrabronchial administration, hypodermic administration, or percutaneous administration. In particular, the adenovirus vector of the present invention exhibits high directivity toward a particular tissue or cell and thus can efficiently deliver a target gene to such a particular tissue or cell. Thus, diagnosis and treatment can be efficiently performed by intravascular administration.

The adenovirus vector of the present invention can be administered in a therapeutically effective amount. A person skilled in the field of gene therapy can readily determine a therapeutically effective amount. The dose can be adequately modified in accordance with pathological severity, sexuality, age, body weight, habit, or other factors of the subject. For example, the adenovirus vector of the present invention can be administered locally into the tumor in an amount of 0.3 × 10¹⁰ to 3 × 10¹² vp (the number of viral particles).

Use of the REIC/Dkk-3 gene or the REIC/Dkk-3 DNA in the present invention encompasses the use of the REIC/Dkk-3 DNA adenovirus vector comprising the REIC/Dkk-3 DNA (Ad-REIC).

Examples of anti-tumor agents to be used in combination with the REIC/Dkk-3 gene include a platinum-containing agent, a tyrosine kinase inhibitor, and an immune checkpoint inhibitor.

Examples of platinum-containing agents include Cisplatin, Carboplatin, Nedaplatin, Oxaliplatin, Miriplatin, Mitaplatin, Satraplatin, Picoplatin, and Triplatin.

Examples of tyrosine kinase inhibitors include Sorafenib, Regorafenib, Lenvatinib, Cabozantinib, Axitinib, Sunitinib, Pazopanib, and Erlotinib. An example of a tyrosine kinase inhibitor is a tyrosine kinase inhibitor that targets the vascular endothelial growth factor receptor (VEGFR). Examples of VEGFR-targeting tyrosine kinase inhibitors include Sorafenib, Regorafenib, Lenvatinib, Cabozantinib, Axitinib, Sunitinib, and Pazopanib, among which Sorafenib, Regorafenib, and Lenvatinib are particularly preferable.

Immune checkpoint inhibitors include anti-PD-1 (programmed cell death 1) antibody, anti-PD-Ll (programmed cell-death ligand 1) antibody, and the like. Immune checkpoint inhibitors are PD-1/PD-L1 immune checkpoint pathway inhibitors, such as anti-PD-1 (programmed cell death 1) antibody and anti-PD-Ll (programmed cell-death ligand 1) antibody. The PD-1/PD-L1 immune checkpoint pathway blocks T cell activation with the aid of immune checkpoint molecules such as PD-1/PD-L1 to downregulate the immune system. Such immune checkpoint molecules are known to promote antigen-specific T cell apoptosis (programmed cell death) in lymph nodes and reduce regulatory T cell (Treg) apoptosis.

With the use of such anti-tumor agents in combination with the REIC/Dkk-3 gene, effects of cancer treatment can be improved to a significant extent. In addition, the amount of chemotherapeutic agents to be used can be reduced to a significant extent. As a result, side effects of anti-tumor agents can be alleviated.

When chemotherapeutic agents are used, cancer cells may exhibit resistance (tolerance) to chemotherapeutic agents. When chemotherapeutic agents are used in combination with the REIC/Dkk-3 gene, cancer cells can be effectively suppressed from developing tolerance to chemotherapeutic agents.

The doses of anti-tumor agents vary depending on symptoms, age, body weight, and other conditions. A daily dose of 0.001 mg to 100 mg may be administered in a single instance or several separate instances a day at intervals of several days, several weeks, or several months.

The REIC/Dkk-3 protein encoded by the REIC/Dkk-3 gene upregulates the anti-cancer immune system, so that cancer can be treated or prevented. In addition, cancer cell apoptosis is induced. Specifically, the REIC/Dkk-3 protein induces cytotoxic T lymphocytes (CTLs) and CTLs attack cancer cells throughout the body.

The REIC/Dkk-3 gene and the anti-tumor agent exert synergistic effects in cancer treatment. Use of the REIC/Dkk-3 gene in combination with the anti-tumor agent is more effective for cancer treatment than the use of the REIC/Dkk-3 gene or the anti-tumor agent by itself.

Administration of the REIC/Dkk-3 gene can be performed simultaneously, separately, or continuously with administration of the anti-tumor agent. Administration of the REIC/Dkk-3 gene can be performed before or after administration of the anti-tumor agent. The REIC/Dkk-3 gene is preferably administered simultaneously with the anti-tumor agent. In the case of simultaneous administration, they can be administered in the form of separate preparations or in the form of an anti-tumor composition containing both the REIC/Dkk-3 gene and the anti-tumor agent. When the REIC/Dkk-3 gene is administered separately from the anti-tumor agent, the anti-tumor agent is administered 1 to 24 hours before or 1 to 30 days before or after the REIC/Dkk-3 gene is administered. Also, the anti-tumor agent and the REIC/Dkk-3 gene can be administered at the same intervals. When the REIC/Dkk-3 gene is administered a plurality of instances, the anti-tumor agent is administered once. When the anti-tumor agent is administered a plurality of instances, alternatively, the REIC/Dkk-3 gene is administered once.

There is no limitation of the administration route of the REIC/Dkk-3 gene,-or the anti-tumor agent, or a composition containing the REIC/Dkk-3 gene and the anti-tumor agent. The administration thereof can be made via hypodermic, intramuscular, or intravenous injection. A systemic or local administration agent may be used. Preferably, the REIC/Dkk-3 gene is a local administration agent targeting a cancer site, and the anti-tumor agent to be used in combination is a systemic or local administration agent. A platinum-containing agent, such as Cisplatin, is preferably an agent for intravenous injection.

The REIC/Dkk-3 gene, the anti-tumor agent, or a composition containing the REIC/Dkk-3 gene and the anti-tumor agent contains a carrier, a diluent, and an excipient, which are usually used in the pharmaceutical field. Examples of a carrier and an excipient used for tablets include monosaccharides, such as glucose, galactose, mannose, and fructose (including sugar alcohols, such as erythritol, xylitol, sorbitol, and mannitol), disaccharides, such as lactose, maltose, sucrose, and trehalose (including sugar alcohols, such as lactitol and maltitol), oligosaccharides, polysaccharides (including thickening polysaccharides, starch, powdered cellulose, microcrystalline cellulose, dextrin, dextran, maltodextrin, and isomaltodextrin), calcium carbonate, kaolin, dibasic calcium phosphate, tribasic calcium phosphate, calcium sulfate, and magnesium stearate. Examples of aqueous liquids used for injection include physiological saline and isotonic solutions containing saccharides such as glucose or other adjuvants, which may be used in combination with adequate solubilizers, including alcohols, polyalcohols such as propylene glycol, and nonionic surfactants. Examples of oily liquids that can be used include sesame oil and soybean oil. Examples of solubilizers that may be used in combination include benzyl benzoate and benzyl alcohol. The carrier, the diluent, and the excipient can be used independently of each other or in adequate combinations of two or more.

Hepatic cancer is a therapeutic target of the present invention. Hepatic cancer encompasses primary hepatic cancer and metastatic hepatic cancer.

The present invention includes a composition comprising the REIC/Dkk-3 gene in combination with the anti-tumor agent, a combinatorial therapeutic agent, and a kit of combinatorial therapeutic agents.

The present invention also includes a method of using the REIC/Dkk-3 gene in combination with the anti-tumor agent when preparing a pharmaceutical product used for cancer treatment.

The present invention also includes a therapeutic agent comprising the REIC/Dkk-3 gene in combination with the anti-tumor agent.

The present invention further includes the REIC/Dkk-3 gene to be used in combination with the anti-tumor agent.

### Examples

The present invention is described in greater detail with reference to the following examples, although the present invention is not limited to these examples.

### [Example 1] Tumor growth inhibition (in vitro) with the use of the REIC/Dkk-3 in combination with Cisplatin

### <Materials and method>

### Cell lines and cell culture:

The hepatic cancer cell lines Hep3B and Huh7 (hereafter, referred to as "Hep3B cells" and " Huh7 cells," respectively, according to need) were purchased from the Japanese Cancer Resources Bank (Tokyo, Japan). The cells were maintained in Dulbecco's Modified Eagle's Medium supplemented with 10% FBS, 1% non-essential amino acid, 1% penicillin/streptomycin, and amphotericin B (0.5 µg/ml). The cells were cultured in accordance with a conventional technique in a humidified 5% CO₂ incubator at 37°C. When the cells reached subconfluency, they were pretreated in a medium containing 0.1% dialyzed FBS for 24 hours before experimentation and then subjected to experimentation.

### Construction and production of adenovirus vector

Full-length complementary DNA of the human REIC/Dkk-3 gene was integrated into a cosmid vector pAxCAwt, and the vector was then transferred into an adenovirus vector in accordance with the COS-TPC method (Takara Bio, Shiga, Japan). Subsequently, 3 enhancers; i.e., hTERT, SV40, and CMV, were added to a site downstream of the poly A sequence of the inserted REIC/Dkk-3 gene to obtain an adenovirus vector comprising the REIC/Dkk-3 gene introduced thereinto (Ad-REIC). As a control vector, an adenovirus vector comprising the LacZ gene introduced thereinto (Ad-LacZ) was used.

### <REIC protein expression in hepatic cancer cell lines>

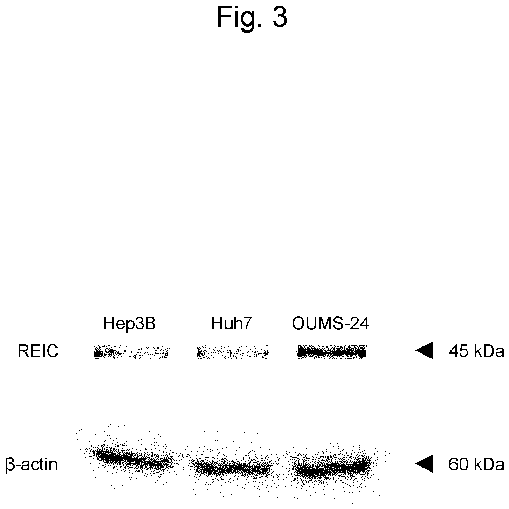

REIC protein expression was evaluated using the hepatic cancer cell lines (Hep3B cells and Huh7 cells), and OUMS-24 cells were used as positive control cells. REIC expression was detected by immunoblotting. Immunoblotting of β-actin was performed to confirm equal loading of proteins in the cells. The results are shown in Fig. 3. As shown in Fig. 3, REIC expression was disappeared or attenuated in all hepatic cancer cell lines.

### <Activity of Ad-REIC to lower cell viability of hepatic cancer cell lines>

The hepatic cancer cell lines Hep3B and Huh7 were cultured in the presence or absence of Ad-LacZ or Ad-REIC for 72 hours. Cell viability was evaluated via MTT assays. Specifically, cells (2 × 10⁵ cells) were seeded on a flat-bottomed 6-well plastic tissue culture plate and cultured for 24 hours in accordance with a conventional technique. The cells were treated in 0.5 ml of a serum-free medium with Ad-LacZ at the multiplicity of infection (MOI) of 50 and Ad-REIC at MOI of 50, respectively, for 1 hour. Thereafter, 1.5 ml of a fresh medium was added. After the cells were cultured for 72 hours, 200 µl of the MTT reagent was added to each well, the cells were then incubated for an additional 4 hours at 37°C. A blue-purple formazan precipitate was dissolved in DMSO, and 100 µl of the solution was transferred to a flat-bottomed 96-well microplate. Mitochondrial activity reflecting cell viability was evaluated by measuring the absorbance at 570 nm using a microplate reader. The results are shown as the mean ± the standard error (n = 5) (^{∗∗} p <0.01).

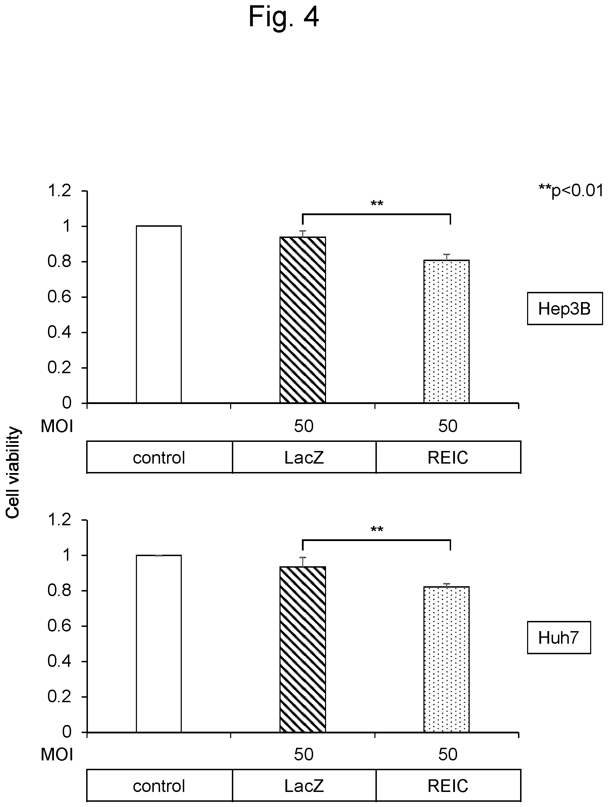

The results are shown in Fig. 4. As shown in Fig. 4, cell viability of hepatic cancer cell lines was lowered upon treatment with Ad-REIC (MOI of 50). The rates of Hep3B and Huh7 cells decreased were 80.8 ± 3.42% and 82.2 ± 1.84%, respectively, compared with the rates attained upon treatment with Ad-LacZ.

### <Activity of Ad-REIC in combination with Cisplatin to lower cell viability of hepatic cancer cell lines>

Hep3B cells and Huh7 cells were cultured in the presence or absence of Cisplatin (1 µg/ml) for 72 hours and in the presence or absence of Ad-Lac Z (MOI of 50) or Ad-REIC (MOI of 50). Cell viability was evaluated by the MTT assays described above. The results are shown as the mean ± the standard error (n = 5) (^{∗∗} p <0.01).

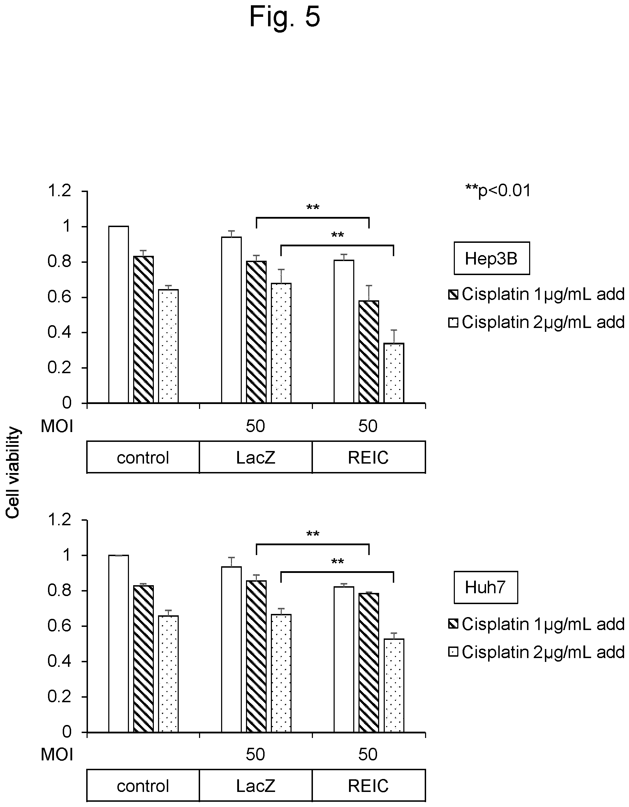

The results are shown in Fig. 5. As shown in Fig. 5, cell viability of hepatic cancer cell lines was lowered upon treatment with Ad-REIC (MOI of 50) and Cisplatin. The rates of the Hep3B cells and Huh7 cells decreased were 58.0 ± 8.57% and 78.6 ± 0.63%, respectively, compared with the rates attained upon treatment with Ad-LacZ ± Cisplatin. In addition, cell viability of the Hep3B cells and that of Huh7 cells were decreased depending on the amount of Cisplatin administered.

### <Apoptosis induction by Ad-REIC through JNK signal transmission pathway>

In the past, REIC overexpression was reported to induce apoptosis by increasing ER stresses in the prostate cancer cell lines, the pancreatic cancer cell lines, and the hepatocellular cancer cell lines (Uchida D., et al., J. Gastroenterol. Hepatol., 2014, 29: 973-983).

The mechanism of Ad-REIC for apoptosis induction was examined. By performing immunoblotting, activation of signal transmission molecules in the JNK signal transmission pathway by Ad-REIC and Cisplatin was evaluated. Ad-LacZ and Ad-REIC at MOI of 10 each were transfected into the Hep3B cells and the Huh7 cells, and cell lysates were collected 48 hours thereafter. Protein expression of phospho-ASK1 (p-ASK1), phospho-IREla (p-IRE1α), phospho-JNK (p-JNK), and phospho-c-Jun (p-c-Jun) β-actin was analyzed via immunoblotting using specific antibodies. Immunoblotting of β-actin was performed to examine equal loading of proteins in the cells.

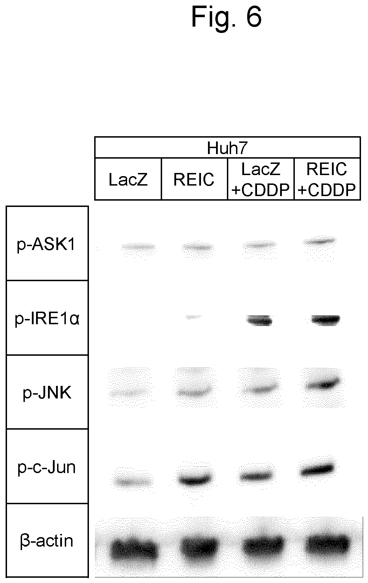

The results are shown in Fig. 6. As shown in Fig. 6, whether or not expression of p-ASK1, p-IRE1α, p-JNK, and p-c-Jun would be activated in response to the treatment with Ad-REIC in hepatic cancer cell line (Huh7) was analyzed by immunoblotting. When Cisplatin was added, each such expression was activated, regardless of the presence or absence of Ad-REIC.

### [Example 2] Tumor growth inhibition in xenograft mouse models by treatment with the REIC/Dkk-3 in combination with Cisplatin

### <Materials and method>

### Experimental animals:

BALB/c-nu/nu female mice (6- to 8-week-old) were purchased from Japan SLC Inc. and maintained with free access to feeds and water in the SPF environment in Okayama University animal experimentation facility. Mice were adapted to the breeding environment over a period of 1 week or longer before the experimentation was initiated. Mice were bred and treated under the strictly controlled conditions in accordance with the guidelines provided by the institutional animal control committee, Okayama University Medical School.

### Tumor growth assays in xenograft mouse models:

In order to determine the therapeutic benefits of Ad-REIC and Cisplatin, xenograft mouse models were constructed using Huh7 cells. Huh7 cells (1.0 × 10⁸ cells, suspended in 200 µl of PBS) were injected hypodermically into the right flanks of BALB/c-nu/nu mice. When the tumor diameters reached approximately 5 to 10 mm, mice were randomly divided into 4 treatment groups each consisting of 5 mice (i.e., the Ad-LacZ-administered group, the Ad-REIC-administered group, the Ad-LacZ + Cisplatin-administered group, and the Ad-REIC + Cisplatin-administered group). The day on which administration was initiated was designated Day 0, and Ad-LacZ (control, 1.0 × 10⁹ pfu) and Ad-REIC (1.0 × 10⁹ pfu) were administered intratumorally on Days 0, 7, and 14. Cisplatin (1 mg/kg) was administered intraperitoneally on Days 0, 7, and 14. Tumor sizes were measured every week, and tumor volumes were determined in accordance with the following equation: V = 1/2 × (shortest diameter) × 1/2 × (shortest diameter) × 1/2 × (longest diameter) × 4/3 × π. In addition, the average tumor volume was determined, and the tumor growth curve was prepared. The results are shown as the mean ± SE (n = 5) (^{∗}p < 0.05, ^{∗∗}p < 0.01).

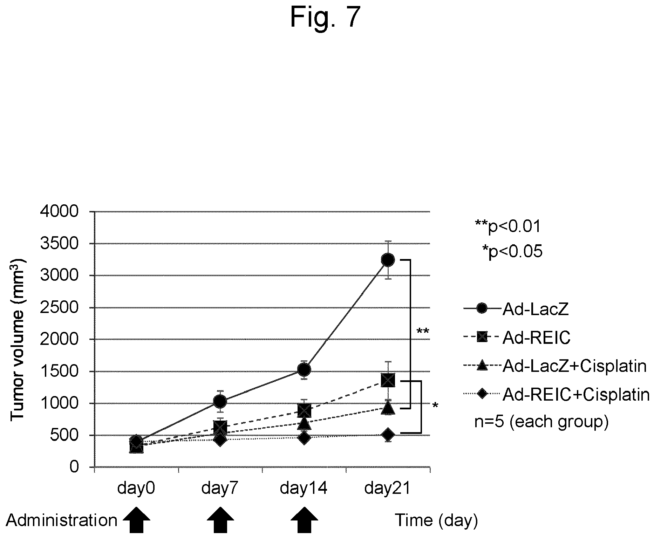

The results are shown in Fig. 7. As shown in Fig. 7, tumors grew progressively in the control Ad-LacZ-administered group. Tumor growth was inhibited in the Ad-REIC-administered group, the Ad-REIC + Cisplatin-administered group, and the Ad-LacZ + Cisplatin-administered group. In particular, tumor growth was inhibited to a significant extent in the Ad-REIC + Cisplatin combination-administered group. In this group, the rate of tumor growth inhibition was 68.7 ± 4.9%, in comparison with the Ad-LacZ + Cisplatin-administered group.

### [Example 3] Tumor growth inhibition in allograft mouse models by treatment with the REIC/Dkk-3 in combination with Cisplatin

### <Materials and method>

### Experimental animals:

C57BL/6 female mice (6- to 8-week-old) were purchased from Japan SLC Inc. and maintained with free access to feeds and water in the SPF environment in Okayama University animal experimentation facility. Mice were adapted to the breeding environment over a period of 1 week or longer before the experimentation was initiated. Mice were bred and treated under the strictly controlled conditions in accordance with the guidelines provided by the institutional animal control committee, Okayama University Medical School.

### Tumor growth assays in allograft mouse models:

The mouse hepatocellular cancer cell line Hep1-6 (2.0 × 10⁸ cells, suspended in 200 µl of PBS) were injected into the right flanks of the C57BL/6 mice (Day 0). When the tumor diameters reached 5 to 10 mm, administration of Ad-REIC and Cisplatin was initiated. Ad-REIC (1.0 × 10⁹pfu) was administered intratumorally on Days 14 and 16. Cisplatin (1 mg/kg) was administered intraperitoneally on Days 14 and 21. Tumor sizes were measured 2 times a week, and tumor volumes were determined.

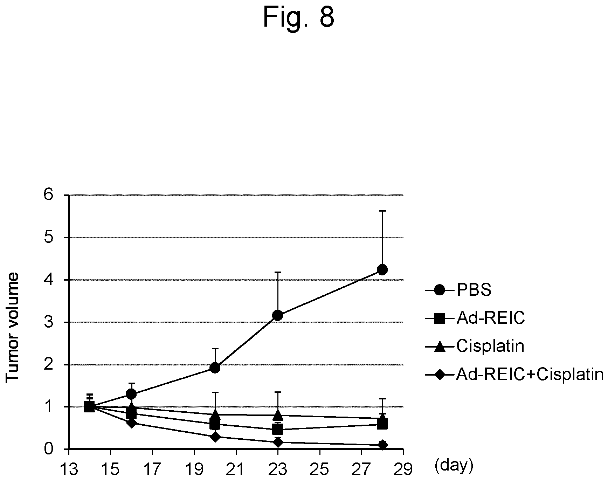

The results are shown in Fig. 8. In the control PBS-administered group, tumors grew progressively. In the Ad-REIC-administered group and in the Cisplatin-administered group, the tumor volume was decreased, and anti-tumor effects were observed. In addition, anti-tumor effects observed in the Ad-REIC + Cisplatin combination-administered group were higher than those observed in the Ad-REIC-administered group and in the Cisplatin-administered group.

### [Example 4] Tumor growth inhibition in allograft mouse models by treatment with the REIC/Dkk-3 in combination with anti-PD-1 antibody

The mouse hepatocellular cancer cell line Hep1-6 (2.0 × 10⁸ cells, suspended in 200 µl of PBS) were injected into the right flanks of the C57BL/6 mice (Day 0). When the tumor diameters reached 5 to 10 mm, administration of Ad-REIC and the anti-PD-1 antibody was initiated. Ad-REIC (1.0 × 10⁹ pfu) was administered intratumorally on Days 14 and 16. The anti-PD-1 antibody (10 mg/kg) was administered intraperitoneally on Day 21. Tumor sizes were measured 2 times a week, and tumor volumes were determined.

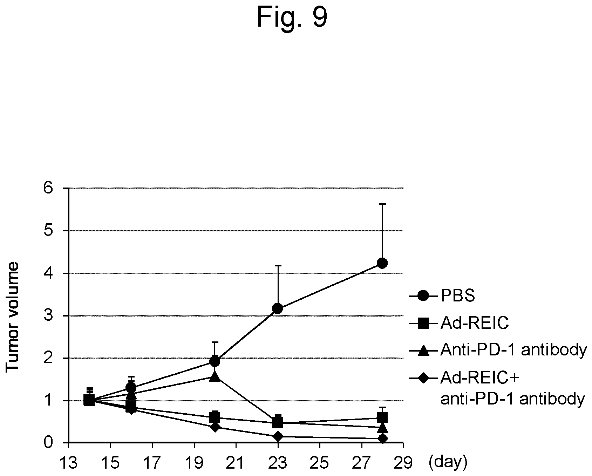

The results are shown in Fig. 9. In the control PBS-administered group, tumors grew progressively. In the Ad-REIC-administered group and in the anti-PD-1 antibody combination-administered group, the tumor volume was decreased to reveal anti-tumor effects. In addition, anti-tumor effects observed in the Ad-REIC + anti-PD-1 antibody-administered group were higher than those observed in the Ad-REIC-administered group and in the anti-PD-1 antibody-administered group.

### [Example 5] Tumor growth inhibition in allograft mouse models by treatment with the REIC/Dkk-3 in combination with tyrosine kinase inhibitor

The mouse hepatocellular cancer cell line Hep1-6 (2.0 × 10⁸ cells, suspended in 200 µl of PBS) were injected into the right flank of the C57BL/6 mice (Day 0). When the tumor diameters reached 5 to 10 mm, administration of Ad-REIC and the tyrosine kinase inhibitor (TKI) was initiated. Ad-REIC (1.0 × 10⁹ pfu) was administered intratumorally on Days 14 and 16. TKI (Sorafenib: 25 mg/kg, Lenvatinib: 1.5 mg/kg) was administered intraperitoneally on Days 14, 16, 21, and 23. Tumor sizes were measured 2 times a week, and tumor volumes were determined.

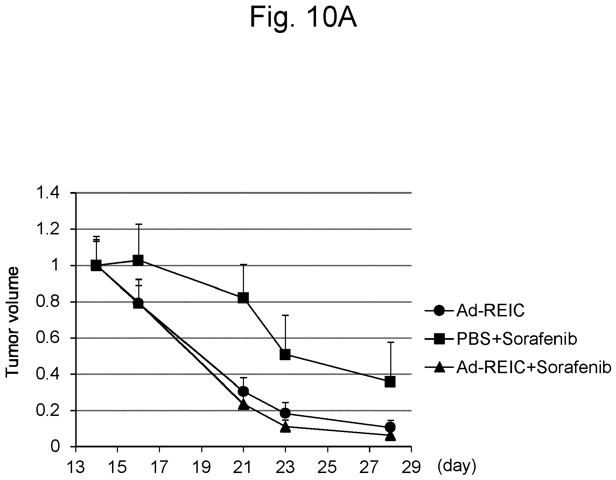

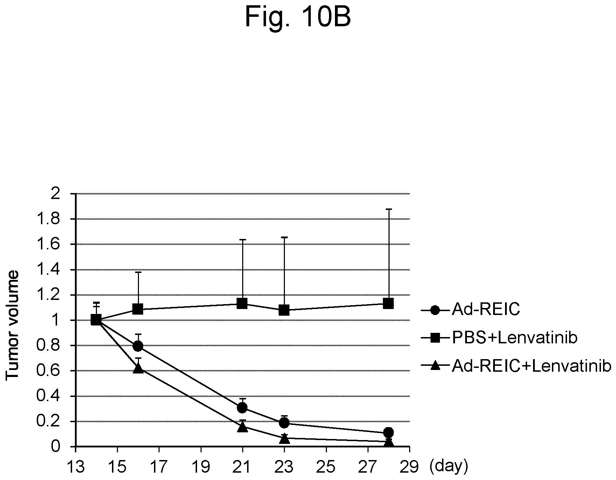

The results are shown in Fig. 10A (Sorafenib) and Fig. 10B (Lenvatinib). In the control PBS-administered group, tumors grew progressively. Anti-tumor effects observed in the Ad-REIC + TKI-administered group were higher than those observed in the Ad-REIC-administered group and in the TKI-administered group.

### [Example 6] Tumor growth inhibition (in vitro) by treatment with the REIC/Dkk-3 in combination with tyrosine kinase inhibitor

Hepa1-6 cells were cultured in the presence or absence of TKI (Sorafenib: 1 µg/ml, Regorafenib: 0.2 µg/ml, Lenvatinib: 0.06 µg/ml) for 72 hours and in the presence or absence of Ad-Lac Z (MOI of 50) or Ad-REIC (MOI of 50). Cell viability was evaluated by the MTT assays described above. The results are shown as the mean ± the standard error (n = 9) (^{∗∗} p <0.01).

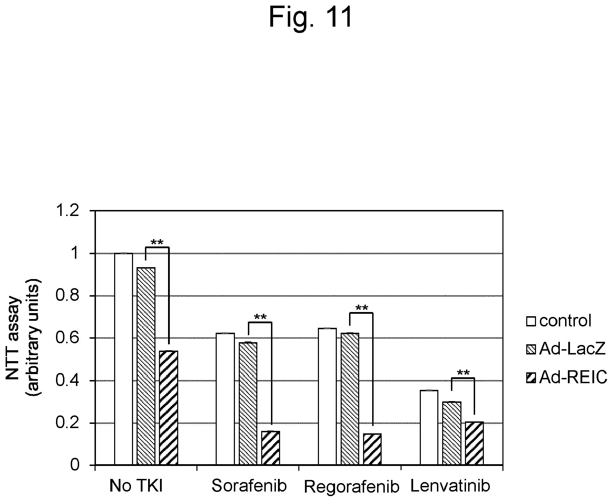

The results are shown in Fig. 11. As shown in Fig. 11, cell viability of hepatic cancer cell lines was lowered upon treatment with Ad-REIC (MOI of 50) and TKI. The cell viability lowered upon treatment with Sorafenib was 72.2 ± 0.1%, that with Regorafenib was 76.2 ± 0.0%, and that with Lenvatinib was 31.8 ± 0.3%, compared with that with Ad-LacZ + TKI.

### Industrial Applicability

Use of the REIC/Dkk-3 gene in combination with the anti-tumor agent is effective as a novel agent for cancer treatment.

All publications, patents, and patent applications cited herein are incorporated herein by reference in their entirety.

## Claims

1. A therapeutic agent for treatment of hepatic cancer used in combination with an anti-tumor agent, which comprises, as an active ingredient, the REIC/Dkk-3 gene.

2. The therapeutic agent according to Claim 1, wherein the REIC/Dkk-3 gene is integrated into an adenovirus vector.

3. The therapeutic agent according to Claim 1 or 2, wherein the anti-tumor agent is a platinum-containing agent.

4. The therapeutic agent according to Claim 3, wherein the platinum-containing agent is selected from the group consisting of Cisplatin, Carboplatin, Nedaplatin, Oxaliplatin, Miriplatin, Mitaplatin, Satraplatin, Picoplatin, and Triplatin.

5. The therapeutic agent according to Claim 1 or 2, wherein the anti-tumor agent is a tyrosine kinase inhibitor.

6. The therapeutic agent according to Claim 5, wherein the tyrosine kinase inhibitor is at least one member selected from the group consisting of Sorafenib, Regorafenib, Lenvatinib, Cabozantinib, Axitinib, Sunitinib, Pazopanib, and Erlotinib.

7. The therapeutic agent according to Claim 5, wherein the tyrosine kinase inhibitor targets a vascular endothelial growth factor receptor (VEGFR).

8. The therapeutic agent according to Claim 7, wherein the VEGFR-targeting tyrosine kinase inhibitor is selected from the group consisting of Sorafenib, Regorafenib, Lenvatinib, Cabozantinib, Axitinib, Sunitinib, and Pazopanib.

9. The therapeutic agent according to Claim 1 or 2, wherein the anti-tumor agent is an immune checkpoint inhibitor.

10. The therapeutic agent according to Claim 9, wherein the immune checkpoint inhibitor is a PD-1/PD-L1 immune checkpoint pathway inhibitor.

11. The therapeutic agent according to any one of Claims 1 to 10, which is in the form of a local administration agent.

12. A kit of combinatorial therapeutic agents for treatment of hepatic cancer, which comprises an anti-tumor agent in combination with the REIC/Dkk-3 gene.

13. The kit of combinatorial therapeutic agents according to Claim 12, wherein the REIC/Dkk-3 gene is integrated into an adenovirus vector.

14. The kit of combinatorial therapeutic agents according to Claim 12 or 13, wherein the anti-tumor agent is a platinum-containing agent.

15. The kit of combinatorial therapeutic agents according to Claim 14, wherein the platinum-containing agent is selected from the group consisting of Cisplatin, Carboplatin, Nedaplatin, Oxaliplatin, Miriplatin, Mitaplatin, Satraplatin, Picoplatin, and Triplatin.

16. The kit of combinatorial therapeutic agents according to Claim 12 or 13, wherein the anti-tumor agent is a tyrosine kinase inhibitor.

17. The kit of combinatorial therapeutic agents according to Claim 16, wherein the tyrosine kinase inhibitor is at least one member selected from the group consisting of Sorafenib, Regorafenib, Lenvatinib, Cabozantinib, Axitinib, Sunitinib, Pazopanib, and Erlotinib.

18. The kit of combinatorial therapeutic agents according to Claim 16, wherein the tyrosine kinase inhibitor targets a vascular endothelial growth factor receptor (VEGFR).

19. The kit of combinatorial therapeutic agents according to Claim 18, wherein the VEGFR-targeting tyrosine kinase inhibitor is selected from the group consisting of Sorafenib, Regorafenib, Lenvatinib, Cabozantinib, Axitinib, Sunitinib, and Pazopanib.

20. The kit of combinatorial therapeutic agents according to Claim 12 or 13, wherein the anti-tumor agent is an immune checkpoint inhibitor.

21. The kit of combinatorial therapeutic agents according to Claim 20, wherein the immune checkpoint inhibitor is a PD-1/PD-L1 immune checkpoint pathway inhibitor.

22. The kit of combinatorial therapeutic agents according to any one of Claims 12 to 21, wherein the REIC/Dkk-3 gene is in the form of a local administration agent.
